Europäisches Patentamt

(19) European Patent Office

Office européen des brevets

(11) Numéro de publication : **0 138 688**
**B1**

(12) FASCICULE DE BREVET EUROPÉEN

(45) Date de publication du fascicule du brevet :
**25.05.88**

(21) Numéro de dépôt : **84401943.0**

(22) Date de dépôt : **28.09.84**

(51) Int. Cl.⁴ : **A 61 L   2/06**

(54) Autoclave et procédé de stérilisation.

(30) Priorité : **03.10.83 FR 8315707**

(43) Date de publication de la demande :
**24.04.85 Bulletin 85/17**

(45) Mention de la délivrance du brevet :
**25.05.88 Bulletin 88/21**

(84) Etats contractants désignés :
**DE FR GB IT SE**

(56) Documents cités :
**BE-A-   658 255**
**FR-A-   940 097 ·**
**FR-A- 1 465 121**
**FR-A- 2 361 934**

(73) Titulaire : **BARRIQUAND, Société dite:**
**9 à 13 Rue Saint Claude**
**F-42300 Roanne (FR)**

(72) Inventeur : **Barriquand, Robert**
**70, rue Roger Salengro**
**F-42300 Roanne (FR)**

(74) Mandataire : **Hasenrader, Hubert et al**
**Cabinet BEAU DE LOMENIE 55, rue d'Amsterdam**
**F-75008 Paris (FR)**

**Description**

La présente invention est relative à des perfectionnements apportés aux autoclaves et aux procédés de stérilisation par un gaz ou un mélange gaz-vapeur, et notamment aux autoclaves de stérilisation à axe horizontal.

Les autoclaves de stérilisation gaz-vapeur sont bien connus dans l'Art antérieur. La stérilisation est réalisée, dans de tels autoclaves, par injection de vapeur dans le gaz présent dans l'autoclave. Dans les cas où il est nécessaire de réguler la pression indépendamment de la température, notamment lorsqu'il est nécessaire de refroidir, dans l'autoclave, les produits stérilisés, cette régulation de pression est réalisée par l'injection ou la purge d'un gaz comprimé, généralement de l'air comprimé.

Toutefois, la présence dans l'autoclave de ce gaz incondensable crée un film entre la vapeur et le produit à stériliser, et diminue de façon sensible le coefficient de transmission de chaleur de la vapeur qui se condense. Pour éliminer cet inconvénient, il a été proposé dans l'Art antérieur de pourvoir ces autoclaves de systèmes à ventilateur qui confèrent au mélange gaz-vapeur une vitesse de circulation propre à améliorer ce coefficient de transmission. Les autoclaves de stérilisation connus de ce type, sont des autoclaves à cuve cylindrique à axe horizontal dont le fond est pourvu d'un ventilateur qui provoque la circulation du mélange gaz-vapeur dans une direction longitudinale. Cependant, de tels systèmes à ventilateur conviennent tout à fait bien essentiellement dans le cas où les autoclaves de stérilisation sont d'une contenance telle qu'ils ne comportent qu'un seul panier de stérilisation. En effet dans le cas d'autoclaves de stérilisation dont la contenance permet la juxtaposition de plusieurs paniers, le mélange gaz-vapeur traverse successivement les paniers juxtaposés, avec une section de passage correspondant à celle d'un panier et une longueur de circulation correspondant à l'addition des longueurs des paniers juxtaposés, ce qui fait que ledit mélange subit une perte de charge dont l'importance est fonction du nombre de paniers traversés, par rapport à ce qu'elle est dans le cas où il n'aurait à traverser qu'un seul panier.

On connaît du document FR 2 361 934 un palliatif à ces inconvénients, selon lequel les espaces latéraux définis entre les paniers et la paroi de l'autoclave sont recoupés par des cloisons séparatrices de manière à définir des canaux « aller » et « retour » longitudinaux, l'air traversant les paniers suivant une direction transversale depuis les canaux « aller » vers les canaux « retour ». Cette solution, n'élimine toutefois pas totalement les inconvénients précités en ce que l'on y maîtrise mal l'homogénéité de l'écoulement transversal du mélange gazeux au travers des paniers en raison de l'apparition de trajets préférentiels, et également en ce que le trajet longitudinal aller et retour du mélange gaz-vapeur entraîne des pertes de charge notables.

Le document FR 1 465 121 illustre une solution dans laquelle le ventilateur aspire le mélange gazeux au niveau de la face supérieure du panier et le refoule latéralement le long d'au moins deux faces latérales opposées, l'air s'écoule ensuite jusqu'à la face inférieure du panier, puis il traverse le panier depuis sa face inférieure jusqu'à sa face supérieure. Les parois latérales de panier sont nécessairement pleines pour éviter des trajets préférentiels depuis les faces latérales en direction de la face supérieure.

Compte tenu de la structure considérée, si l'on cherche à réaliser un appareil capable de contenir plusieurs paniers, on aboutit nécessairement à un appareil où les paniers seraient superposés et présenterait les mêmes inconvénients qu'un autoclave horizontal avec circulation d'air successivement dans les paniers juxtaposés. De plus, l'injection ponctuelle de vapeur conduit à une mauvaise homogénéité du mélange gaz-vapeur.

Enfin, la présence de plusieurs circuits d'écoulement oblige à multiplier les dispositifs annexes de conditionnement et conduit à une perte de charge relativement élevée.

La présente invention s'est en conséquence donné pour but de réaliser un autoclave de stérilisation par un gaz ou un mélange gaz-vapeur, à axe horizontal, qui répond mieux aux nécessités de la pratique que les autoclaves de ce type antérieurement connus, notamment en ce que sa conception est telle que la section de passage du gaz ou du mélange gaz-vapeur n'est pas limitée à une section de passage correspondant à un seul panier et est considérablement augmentée, en ce que la perte de charge n'est pas affectée dans le sens d'une augmentation de sa valeur, par la disposition de plusieurs paniers dans un tel autoclave, et en ce que le débit du gaz ou du mélange gaz-vapeur dans l'autoclave est augmenté dans une mesure importante, sans qu'il soit nécessaire d'augmenter la puissance des moyens d'entraînement du système de ventilateurs par rapport à celle utilisée dans les systèmes connus.

A cet effet, la présente invention propose un autoclave de stérilisation par un gaz ou un mélange gaz-vapeur, comprenant une cuve sensiblement cylindrique à axe horizontal et apte à recevoir plusieurs paniers porte-produits, de forme sensiblement parallèlepipédique, alignés et juxtaposés suivant l'axe de la cuve et présentant de ce fait un premier ensemble de faces latérales d'un premier côté, un ensemble de faces supérieures, et un second ensemble de faces latérales d'un côté opposé, ledit autoclave comportant un dispositif d'injection de vapeur et/ou de conditionnement de gaz et des moyens de circulation de mélange gaz-vapeur, caractérisé en ce que les moyens de circulation comprennent une chambre de ventilateurs s'étendant le long du premier ensemble de faces latérales, fermée en partie basse et ouverte en partie haute, au moins un ventilateur monté dans ladite chambre latéralement par rapport à l'axe de la cuve, l'ouïe d'aspiration dudit ventilateur étant située en regard de la face latérale correspondante d'un panier ou d'un groupe de paniers, un déflecteur horizontal recouvrant l'ensemble des faces supérieures des paniers et définissant avec le sommet de la cuve une chambre de transfert, le second ensemble des faces latérales délimitant avec la paroi de cuve adjacente une chambre d'admission, dont il résulte que l'écoulement d'air s'effectue exclusivement transversalement à l'axe de la cuve sur toute la longueur de celle-ci suivant un circuit fermé unique traversant successivement la chambre de ventilateurs, la chambre de transfert, la chambre d'admission, et enfin les paniers eux-mêmes depuis le second ensemble de faces latérales jusqu'au premier ensemble de faces latérales, ledit dispositif d'injection de vapeur et/ou de conditionnement de gaz s'étendant sur tout le long de la cuve dans la chambre de transfert.

Selon un mode de réalisation avantageux de l'autoclave de stérilisation conforme à l'invention, le dispositif d'injection de vapeur comporte des buses orientées dans le sens de circulation du mélange gaz-vapeur.

Selon un autre mode de réalisation avantageux de l'autoclave de stérilisation conforme à l'invention, celui-ci comporte un ventilateur agencé latéralement en regard de chaque panier.

Selon encore un autre mode de réalisation avantageux de l'autoclave de stérilisation conforme à l'invention, le dispositif d'injection de vapeur est de préférence constitué par une rampe d'injection agencée à proximité et en aval des ventilateurs latéraux, qui alimente simultanément la totalité des paniers de l'autoclave.

Selon un autre mode de réalisation avantageux de l'autoclave de stérilisation conforme à l'invention, celui-ci est équipé d'un échangeur de chaleur, de préférence de type compact, inclus dans ledit autoclave dans la chambre de transfert.

Conformément à un autre mode de réalisation avantageux de l'autoclave de stérilisation conforme à l'invention, un pulvérisateur d'eau est monté en amont des produits à stériliser, dans le sens de la circulation du gaz ou du mélange gaz-vapeur, dans la chambre d'admission.

Selon une disposition avantageuse de ce mode de réalisation, le pulvérisateur d'eau comporte des gicleurs orientés dans le sens de circulation du gaz ou du mélange gaz-vapeur.

L'invention concerne également un procédé de stérilisation de produits dans une pluralité de paniers juxtaposés dans un autoclave de stérilisation ayant une cuve à axe horizontal, dans lequel on conditionne un gaz ou un mélange gaz-vapeur dans la cuve de l'autoclave, et on entraîne en circulation ledit gaz ou mélange gaz-vapeur, caractérisé en ce que l'on entraîne ledit gaz ou mélange en circulation exclusivement transversale à l'axe de la cuve suivant un circuit fermé unique.

Le refroidissement peut-être assuré, conformément à la présente invention, par pulvérisation d'eau froide par le dispositif pulvérisateur précité, qui peut avantageusement être réalisé sous la forme d'une rampe de pulvérisation.

Egalement conformément à l'invention, le refroidissement peut être assuré pendant une première phase par circulation d'eau froide dans l'échangeur et pendant une deuxième phase, consécutive à la première, par circulation dans l'échangeur d'un agent de refroidissement liquide tel que saumure ou eau glycolée notamment.

Selon une disposition avantageuse de l'invention, applicable au refroidissement en deux phases précité, l'autoclave est équipé d'un réservoir tampon extérieur contenant l'agent de refroidissement liquide, associé à un groupe réfrigérant de puissance réduite qui en assure le refroidissement.

Dans le mode de réalisation de l'autoclave de stérilisation dans lequel la stérilisation est assurée uniquement par un gaz, et notamment par de l'air, l'air ou analogue de stérilisation est chauffé et refroidi par circulation dans l'échangeur de chaleur qui assure alternativement son chauffage par vapeur et son refroidissement par de l'eau, puis éventuellement par un fluide réfrigérant liquide tel que saumure, eau glycolée ou analogue.

Outre les dispositions qui précèdent, l'invention comprend encore d'autres dispositions, qui ressortiront de la description qui va suivre.

La présente invention vise plus particulièrement les autoclaves de stérilisation par un gaz ou un mélange gaz-vapeur conformes aux dispositions qui précèdent, ainsi que les chaînes de traitement incluant de tels autoclaves.

L'invention sera mieux comprise à l'aide du complément de description qui va suivre, qui se réfère aux dessins annexés dans lesquels :

la figure 1 est une vue en coupe axiale d'un autoclave de stérilisation conforme à la présente invention, et

la figure 2 est une vue en coupe, à 90° par rapport à la coupe de la figure 1, et à plus grande échelle, de l'autoclave de la figure 1 et des circuits associés.

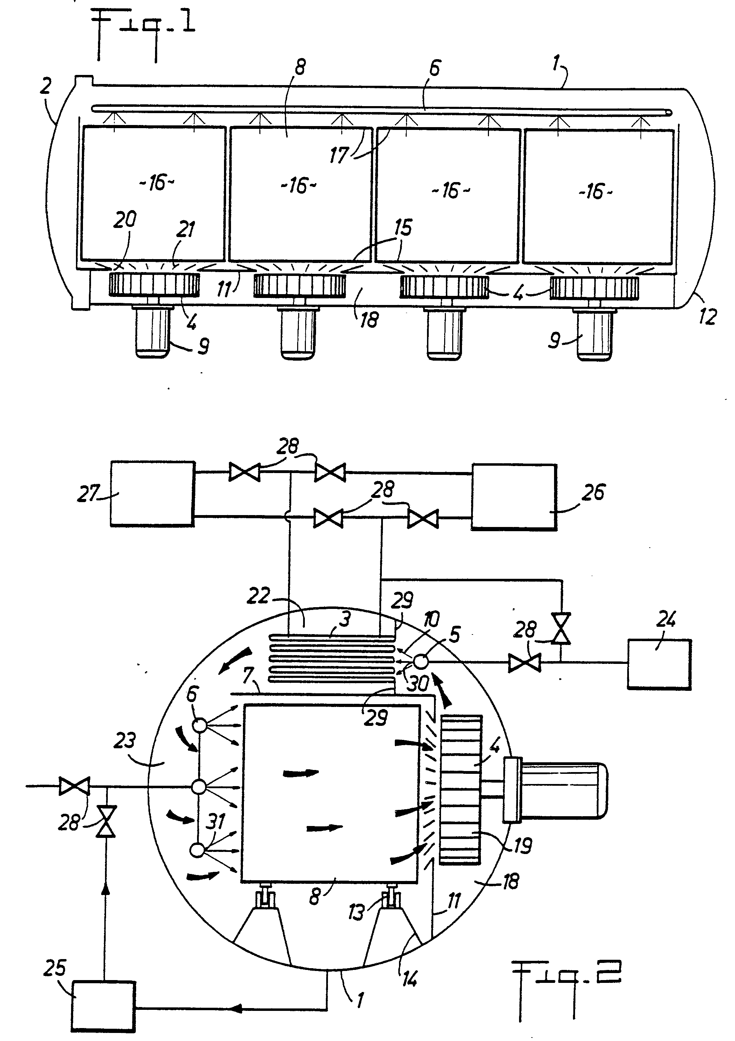

Il doit être bien entendu, toutefois, que ces dessins et les parties descriptives correspondantes, sont donnés uniquement à titre d'illustration de l'objet de l'invention, dont ils ne constituent en aucune manière une limitation.

L'autoclave de stérilisation représenté au dessin comprend une cuve 1 sensiblement cylindrique d'axe horizontal fermée par un couvercle amovible 2 et comportant un fond 12, contenant une pluralité de paniers 8 de forme cubique, qui, dans l'exemple de réalisation représenté, sont au nombre de quatre.

Ces paniers sont, par exemple, réalisés avec une armature à tiges portant un ou plusieurs plateaux ajourés qui sont destinés à recevoir les produits à stériliser.

De cette manière de l'air en circulation peut traverser pratiquement librement les paniers 8. De plus, les paniers sont pourvus de roulettes 13 à leurs bases, qui sont guidées par des rails 14 fixés en partie basse de la cuve. En raison de leur juxtaposition en alignement dans l'axe de la cuve, les paniers présentent un premier ensemble de faces latérales 15 un ensemble de faces supérieures 16 et un second ensemble de faces latérales 17.

Un ventilateur centrifuge 4, entraîné par un moteur 9, est agencé en regard de chacun des paniers 8, suivant un axe perpendiculaire à l'axe de l'autoclave 1.

Une cloison longitudinale 11 s'étend sur toute la longueur de la cuve, le long du premier ensemble de faces latérales des paniers et définit avec la paroi voisine de la cuve une chambre de ventilateurs 18, fermée en partie basse et ouverte en partie haute, dans laquelle sont disposés les rotors à aubes 19 des ventilateurs 4 centrifuges. Les moteurs des ventilateurs sont naturellement disposés à l'extérieur de la cuve pour des raisons que l'on comprendra aisément. Au droit de chaque rotor, et donc de chaque panier, la cloison est percée d'une ouïe circulaire 20, et en amont de celle-ci est disposé un diffuseur 21 raccordé à l'ouïe et s'étendant sur toute la section d'un panier, ce qui permet de répartir l'aspiration du ventilateur sur toute la section du panier et éviter la stagnation de gaz dans les coins du panier.

L'on comprendra aisément que la disposition d'un ventilateur par panier, est particulièrement avantageuse pour des raisons qui seront précisées plus loin ; on pourra cependant prévoir, par exemple, un ventilateur pour deux paniers, tout en maintenant la disposition latérale des ventilateurs.

De plus un déflecteur horizontal 7, placé au dessus de l'ensemble des faces supérieures des paniers et raccordé au sommet de la cloison 11 forme avec le sommet de la cuve une chambre de transfert 22 qui guide la circulation du mélange air vapeur depuis le haut de la chambre 18 jusqu'à la chambre 23 délimitée entre la cuve et le second ensemble de faces latérales, dite chambre d'admission.

Une rampe d'injection de vapeur 5 est agencée immédiatement en aval des ventilateurs 4 et au début de la chambre de transfert 22, donc en amont des produits à stériliser contenus dans les paniers 8, un tel agencement étant propre à assurer un brassage optimal du mélange air-vapeur avant l'entrée en contact de ce dernier avec les produits à stériliser.

De plus, la rampe d'injection de vapeur 5 est équipée de buses 30 orientées de manière à injecter la vapeur dans le sens de circulation du mélange air-vapeur, ce qui permet d'obtenir un effet d'entraînement, ou effet de trompe, dans le sens de la circulation du mélange air-vapeur, représenté par les flèches 10 ; cet effet d'entraînement améliore le débit du mélange air-vapeur.

Une rampe 6 de pulvérisation de fluide de refroidissement est montée en amont des produits à stériliser dans la chambre d'admission 23 ; son agencement et l'orientation de ses gicleurs 31 est telle que le fluide de refroidissement est pulvérisé dans le sens de circulation du mélange air-vapeur.

Un échangeur de chaleur 3, de préférence de type compact, est monté à l'intérieur de la cuve de l'autoclave 1, entre le déflecteur 7 et le sommet de la cuve, dans la chambre de transfert 22. Il est avantageux que l'échangeur soit agencé suivant un axe parallèle à l'axe de l'autoclave ; il est également avantageux que l'échangeur soit disposé immédiatement en aval de la rampe 5 d'injection de vapeur, dans le sens de circulation du mélange air-vapeur. En effet, la présence de l'échangeur provoque un écoulement turbulent du mélange gaz-vapeur, à la traversée de l'échangeur, qui favorise l'homogénéisation du mélange.

L'échangeur 3 occupe avantageusement la totalité de la section de la chambre de transfert 22, au besoin en prévoyant des plaques d'obturation 29 vers le déflecteur 7 et vers le sommet de la cuve.

Dans un appareil dépourvu d'échangeur, on pourra prévoir un dispositif statique pour améliorer l'homogénéisation.

Le fond du panier est également ajouré, ce qui permet de recueillir aisément au bas de la cuve les gouttes d'eau de condensation qui se forment sur les produits.

Avantageusement, dans la mesure où cette eau de condensation est pure et stérile, elle est collectée dans un réservoir 25 et réutilisée comme eau de pulvérisation dans la rampe 6, ce qui évite de contaminer les produits et/ou leurs enveloppes au cours du refroidissement et d'entartrer les gicleurs de la rampe de pulvérisation 6.

L'agencement latéral des ventilateurs, en combinaison avec la disposition d'injecteurs de vapeur en aval des ventilateurs et à proximité de ces derniers, orientés dans le sens de circulation du mélange air-vapeur, ces injecteurs étant eux-mêmes en amont des produits à stériliser, et avec la disposition et l'orientation de pulvérisateurs d'eau de refroidissement également dans le sens de circulation du mélange air-vapeur, a pour rôle de réaliser un circuit aussi court que possible du mélange air-vapeur, dans lequel la circulation du gaz ou du mélange s'effectue exclusivement transversalement suivant un circuit fermé unique, ce qui améliore considérablement les performances de l'autoclave conforme à l'invention par rapport à celles de l'Art antérieur. En effet, l'agencement relatif des ventilateurs, des injecteurs de vapeur, ainsi que des pulvérisateurs d'eau, conforme à la présente invention, permet d'avoir un débit air-vapeur plus important, avec une même puissance ; si l'on considère le cas d'un autoclave horizontal de contenance 4 paniers de forme cubique, comportant un ventilateur disposé dans l'axe de l'autoclave, sur la porte ou sur le fond, le mélange gaz-vapeur traverse successivement les quatre paniers, la section de passage correspondant à celle d'un panier et la perte de charge est quatre fois plus importante que pour traverser un seul panier.

Contrairement à cela, dans l'autoclave conforme à l'invention, si la vitesse de circulation du mélange gaz-vapeur à travers les paniers est la même que ci-dessus, la longueur de circulation est quatre fois plus petite et, par conséquent, la perte de charge est quatre fois plus faible, tandis que la section de passage est quatre fois plus importante puisqu'elle correspond aux quatre paniers côte-à-côte, ce qui donne un débit quatre fois plus grand. La puissance globale étant proportionnelle au débit et à la perte de charge, elle est donc la même pour un débit quatre fois plus important, si la perte de charge est quatre fois plus faible, et ceci quel que soit le nombre de ventilateurs.

Il est avantageux d'injecter de la vapeur vive par la rampe d'injection 5 à partir d'une source 24 à température élevée et sous pression, pendant la période de montée en température, pour assurer un chauffage homogène dans l'autoclave : le coefficient d'échange et la capacité calorifique sont optimum. L'échangeur 3 peut être utilisé soit en préchauffage, soit en réchauffage avec la vapeur depuis la source 24 pour assurer le chauffage et/ou le maintien en température des produits, par brassage du mélange air-vapeur, qui se trouve alors surchauffé, donc sans provoquer de condensation sur les produits à stériliser, ce qui est particulièrement intéressant dans le cas où les produits à stériliser sont contenus dans des poches en matière plastique souple, car cette technique de maintien en température évite que les poches ne soient mouillées et ne s'opacifient.

De même, le refroidissement par pulvérisation au moyen du pulvérisateur 6 a lieu dans le sens de la circulation du mélange, améliorant ainsi le débit brassé, et donc l'homogénéité du mélange et l'efficacité de l'évacuation de chaleur. De plus, conformément à l'invention, le refroidissement peut être réalisé au moyen de l'échangeur 3 en deux phases, une première phase, par circulation d'eau froide dans l'échangeur 3, à partir d'une première source 26, qui permet d'assurer un refroidissement jusqu'à une température inférieure à 90°, de l'ordre de 70 °C à 80 °C, et une deuxième phase, par circulation dans l'échangeur 3, d'un fluide réfrigérant liquide tel que de la saumure ou de l'eau glycolée à — 15 °C, pour abaisser la température des produits au-dessous de 50°. Le fluide réfrigérant liquide est avantageusement amené à l'échangeur à partir d'un réservoir tampon 27 refroidi par un groupe réfrigérant (non représenté) de puissance réduite puisque le fluide réfrigérant liquide n'est nécessaire que pendant une fraction du cycle de traitement, alors que le groupe réfrigérant peut fonctionner en permanence.

Il a été question dans ce qui précède d'un mélange air-vapeur. L'on comprendra cependant aisément que l'invention n'est pas limitée à l'utilisation de l'air, mais s'étend à tout autre gaz approprié et qu'elle n'est pas limitée à la stérilisation par un mélange gaz-vapeur, la stérilisation par un gaz, sans apport de vapeur, et notamment par de l'air étant également incluse dans le cadre de la présente invention.

Dans le cas où la stérilisation est réalisée à l'aide d'un gaz de stérilisation tel que de l'air par exemple, l'échangeur de chaleur 3 constitue un dispositif de conditionnement qui assure successivement le chauffage de l'air qui traverse l'échangeur, par échange avec de la vapeur qui circule dans ledit échangeur, et son refroidissement par échange avec de l'eau froide, qui circule dans l'échangeur, suivi, éventuellement, d'un refroidissement par échange avec de la saumure, de l'eau glycolée ou analogue qui circule dans l'échangeur.

On notera que l'absence d'équipements annexes aux deux extrémités de la cuve permet de réaliser très facilement un autoclave à deux portes. Dans ce cas le fond 12 de la cuve est remplacée par une porte analogue à la porte 2. Ceci favorise l'accessibilité de l'autoclave.

Bien entendu, les raccordements des différents dispositifs, rampe d'injection de vapeur, échangeur, rampe de pulvérisation d'eau, aux différentes sources qui les alimentent sont commandés à l'aide de vannes 28 de tout type approprié.

**Revendications**

1. Autoclave de stérilisation par un gaz ou un mélange gaz-vapeur, comprenant une cuve (1) sensiblement cylindrique à axe horizontal et apte à recevoir plusieurs paniers (8) porte-produits, de forme sensiblement parallélepipédique, alignés et juxtaposés suivant l'axe de la cuve et présentant de ce fait un premier ensemble de faces latérales (15) d'un premier côté, un ensemble de faces supérieures (16), et un second ensemble de faces latérales (17) d'un côté opposé, ledit autoclave comprenant également un dispositif d'injection de vapeur (5) et/ou de conditionnement de gaz (3) et des moyens de circulation de mélange gaz-vapeur, caractérisé en ce que les moyens de circulation comprennent une chambre de ventilateurs (18) s'étendant le long du premier ensemble de faces latérales, fermée en partie basse et ouverte en partie haute, au moins un ventilateur (4) par groupe de paniers monté dans ladite chambre latéralement par rapport à l'axe de la cuve, l'ouïe d'aspiration (20) dudit ventilateur étant située en regard de la face latérale correspondante d'un panier ou d'un groupe de paniers, un déflecteur horizontal (7) recouvrant l'ensemble des faces supérieures des paniers et définissant avec le sommet de la cuve une chambre de transfert (22), le second ensemble des faces latérales délimitant avec la paroi de cuve adjacente une chambre d'admission (23), dont il résulte que l'écoulement d'air s'effectue exlusivement transversalement à l'axe de la cuve sur toute la longueur de celle-ci suivant un circuit fermé unique traversant successivement la chambre de ventilateurs, la chambre de transfert, la chambre d'admission, et enfin les paniers eux-mêmes depuis le second ensemble de faces latérales jusqu'au premier ensemble de faces latérales, ledit dispositif d'injection de vapeur et/ou de conditionnement de gaz s'étendant sur tout le long de la cuve dans la chambre de transfert (22).

2. Autoclave selon la revendication 1, caractérisé en ce que le dispositif d'injection de vapeur (5) comporte des buses orientées dans le sens de circulation du mélange gaz-vapeur.

3. Autoclave selon l'une des revendications 1 et 2, caractérisé en ce qu'il comporte un ventilateur (4) agencé latéralement en regard de chaque panier (8).

4. Autoclave selon l'une quelconque des revendications 1 à 3, caractérisé en ce que le dispositif d'injection de vapeur (5) est de préférence constitué par une rampe d'injection agencée à proximité et en aval des ventilateurs latéraux (4), qui alimente simultanément la totalité des paniers (8) de l'autoclave.

5. Autoclave selon l'une quelconque des revendications 1 à 4, caractérisé en ce qu'il est équipé d'un échangeur de chaleur (3), inclus dans ledit autoclave dans ladite chambre de transfert (22) en tant que dispositif de conditionnement de gaz.

6. Autoclave selon l'une quelconque des revendications 1 à 5, caractérisé en ce qu'il comprend un pulvérisateur d'eau (6) monté en amont des produits à stériliser, dans le sens de circulation du gaz ou du mélange gaz-vapeur, dans la chambre d'admission (23).

7. Autoclave selon la revendication 6, caractérisé en ce que le pulvérisateur d'eau (6) comporte des gicleurs orientés dans le sens de circulation du gaz ou du mélange gaz-vapeur.

8. Autoclave selon l'une quelconque des revendications 1 à 7, caractérisé en ce qu'il est équipé d'un réservoir tampon (27) extérieur contenant un agent réfrigérant liquide maintenu à basse température, de l'ordre de — 10 °C à — 25 °C, par un groupe réfrigérant de puissance réduite associé audit réservoir tampon.

9. Procédé de stérilisation de produits dans une pluralité de paniers juxtaposés dans un autoclave de stérilisation, selon l'une quelconque des revendications 1 à 8, par gaz ou mélange gaz-vapeur ayant une cuve à axe horizontal, dans lequel on conditionne un gaz ou un mélange gaz-vapeur dans la cuve de l'autoclave, et on entraîne en circulation ledit gaz ou mélange gaz-vapeur, caractérisé en ce que l'on entraîne ledit gaz ou mélange en circulation exclusivement transversale à l'axe de la cuve suivant un circuit fermé unique.

10. Procédé selon la revendication 9, caractérisé en ce que l'on forme un mélange gaz-vapeur dans ladite cuve par injection de vapeur dans le sens de circulation.

11. Procédé selon l'une des revendications 9 à 10, caractérisé en ce que l'on réchauffe ledit gaz ou mélange gaz-vapeur au moyen d'un échangeur placé dans ledit circuit.

12. Procédé de stérilisation selon l'une quelconque des revendications 9 à 11, caractérisé en outre par le fait qu'on assure un refroidissement par pulvérisation d'eau froide orientée horizontalement dans le sens de circulation du mélange gaz-vapeur, en amont des produits à stériliser.

**Claims**

1. Sterilization autoclave of the type using a gas or gas-steam mixture comprising a substantially cylindrical vessel (1) of horizontal axis, capable of receiving a plurality of containers (8) holding products, containers of substantially parallelepipedal shape, juxtaposed in direct line with the axis of the vessel and presenting as a result a first set of lateral faces (15) on a first side, one set of upper faces (16), and a second set of lateral faces (17) on the opposite side, said autoclave further comprising a steam-injecting (5) and/or gastreating (3) device and means causing the gas or gas-steam mixture to flow, characterized in that the flowing means comprise a fan chamber (18) which extends along the first set of lateral faces and which is closed at its lower part and open at its upper part, at least one fan (4) for every group of containers mounted in said chamber laterally with respect to the axis of the vessel, the suction orifice (20) of said fan being situated opposite the corresponding lateral face of a container or of a group of containers, a horizontal deflector (7) covering up all the upper faces of the containers and defining with the top of the vessel a transfer chamber (22), the second set of lateral faces defining an admission chamber (23), with the adjacent wall of the vessel, as a result of which the air flow is directed exclusively transversely to the axis of the vessel throughout the length thereof, following a single closed circuit going through successively the fans chamber, the transfer chamber, the admission chamber and finally the containers proper, from the second set of lateral faces to the first set of lateral faces, the said steam injecting or gas-treating device extending over the full length of the vessel within the transfer chamber (22).

2. Autoclave according to claim 1, characterized in that said steam injection device (5) comprises nozzles oriented in the direction of flow of the gas-steam mixture.

3. Autoclave according to claim 1, characterized in that it comprises a fan (4) disposed laterally in facing relation to each container (8).

4. Autoclave according to any one of claims 1 to 3, characterized in that said steam injection device (5) is preferably constituted by an injection ramp fitted proximate to and downstream of the lateral fans (4), which ramp simultaneously supplies all the containers (8) of the autoclave.

5. Autoclave according to any one of claims 1 to 4, characterized in that said autoclave is equipped with a heat exchanger (3) situated in the autoclave inside said transfer chamber (22) as a gas treating device.

6. Autoclave according to any one of claims 1 to 5, characterized in that it comprises water spraying means (6) mounted upstream of the products to be sterilized, in the direction of flow of the gas or gas-steam mixture, in the admission chamber (23).

7. Autoclave according to claim 6, characterized in that the water-spraying means (6) comprises spraying nozzles directed in the same way as the flow of gas or of gas-steam mixture.

8. Autoclave according to any one of claims 1 to 7, characterized in that said autoclave is equipped with an external buffer reservoir (27) containing a liquid cooling agent kept at low temperature, between — 10 ºC and — 25 ºC by a reduced power cooling unit associated to said buffer reservoir.

9. Method of sterilizing products in a plurality of containers juxtaposed inside an autoclave according to any one of claims 1 to 8, by a gas-steam mixture, having a vessel of horizontal axis, in which a gas or a gas-steam mixture is treated in the vessel of the autoclave, and said gas or gas-steam mixture is caused to flow, characterized in that said gas or gas-steam mixture is caused to flow exclusively in a direction which is transversal to the axis of the vessel and follows a single closed circuit.

10. Method according to claim 9, characterized in that a gas-steam mixture is formed in said vessel by injecting steam in the flowing direction.

11. Method according to any one of claims 9 to 10, characterized in that said gas or gas-steam mixture is re-heated by means of an exchanger placed in said circuit.

12. Method according to any one of claims 9 to 11, characterized in that cooling is achieved by horizontally oriented spraying cold water in the direction of flow of the gas-steam mixture, upstream of the products to be sterilized.

## Patentansprüche

1. Autoklav zur Sterilisation durch ein Gas oder eine Gas/Dampf-Mischung, aufweisend einen Behälter (1) mit horizontaler Achse, der im wesentlichen zylindrisch und geeignet ist, mehrere Produkthalter-Körbe (8) von im wesentlichen Parallelepipedform aufzunehmen, die ausgerichtet und nebeneinander angeordnet folgend der Achse des Behälters sind und von daher einen ersten Satz von Seitenwänden (15) auf einer ersten Seite, einen Satz von oberen Wänden (16) und einen zweiten Satz von Seitenwänden (17) auf einer gegenüberliegenden Seite aufweisen, wobei der Autoklav ebenso eine Vorrichtung zur Zuführung von Dampf (5) und/oder zur Behandlung von Gas (3) und Mittel zur Zirkulation von Gas/Dampf-Mischung aufweist, dadurch gekennzeichnet, daß die Zirkulationsmittel eine sich längs des ersten Satzes von Seitenwänden erstreckende, im unteren Teil geschlossene und im oberen Teil offene Ventilatorenkammer (18), wenigstens einen Ventilator (4) pro Gruppe von Körben, der in der Kammer seitlich in bezug auf die Achse des Behälters angeordnet ist, wobei die Ansaugöffnung (20) des Ventilators gegenüber der entsprechenden Seitenwand eines Korbes oder einer Gruppe von Körben liegt, eine horizontale Ablenkeinrichtung (7) aufweisen, die den Satz von oberen Wänden der Körbe überdeckt und mit der Oberseite des Behälters eine Überführungskammer (22) abgrenzt, wobei der zweite Satz der Seitenwände mit der benachbarten Behälterwand eine Einlaßkammer (23) abgrenzt, woraus sich ergibt, daß die Luftströmung ausschließlich in Querrichtung zur Achse des Behälters über dessen gesamte Länge folgend einem einzigen geschlossenen Kreislauf erfolgt, wobei sie nacheinander die Ventilatorenkammer, die Überführungskammer, die Einlaßkammer und schließlich die Körbe selbst von dem zweiten Satz von Seitenwänden an bis zum ersten Satz von Seitenwänden durchquert, wobei die Vorrichtung zur Zuführung von Dampf und/oder zur Behandlung von Gas sich über die gesamte Länge des Behälters in der Überführungskammer (22) erstreckt.

2. Autoklav nach Anspruch 1, dadurch gekennzeichnet, daß die Vorrichtung zur Zuführung von Dampf (5) Düsen umfaßt, die in der Zirkulationsrichtung der Gas/Dampf-Mischung orientiert sind.

3. Autoklav nach einem der Ansprüche 1 und 2, dadurch gekennzeichnet, daß er einen Ventilator (4) umfaßt, der seitlich gegenüber jedem Korb (8) angeordnet ist.

4. Autoklav nach einem beliebigen der Ansprüche 1 bis 3, dadurch gekennzeichnet, daß die Vorrichtung zur Zuführung von Dampf (5) vorzugsweise durch eine in der Nähe von und stromabwärts der seitlichen Ventilatoren (4) angeordnete Zuführungsrampe gebildet ist, die gleichzeitig die Gesamtheit der Körbe (8) des Autoklaven speist.

5. Autoklav nach einem beliebigen der Ansprüche 1 bis 4, dadurch gekennzeichnet, daß er mit einem Wärmeaustauscher (3) ausgestattet ist, der in dem Autoklaven in der Überführungskammer (22) als Vorrichtung zur Behandlung von Gas enthalten ist.

6. Autoklav nach einem beliebigen der Ansprüche 1 bis 5, dadurch gekennzeichnet, daß er einen Wasserzerstäuber (6) aufweist, der stromaufwärts der zu sterilisierenden Produkte in der Zirkulationsrichtung des Gases oder der Gas/Dampf-Mischung in der Einlaßkammer (23) angebracht ist.

7. Autoklav nach Anspruch 6, dadurch gekennzeichnet, daß der Wasserzerstäuber (6) Spritzdüsen umfaßt, die in der Zirkulationsrichtung des Gases oder der Gas/Dampf-Mischung orientiert sind.

8. Autoklav nach einem beliebigen der Ansprüche 1 bis 7, dadurch gekennzeichnet, daß er mit einem äußeren Pufferreservoir (27) ausgestattet ist, das ein durch eine dem Pufferreservoir zugeordnete Kühlergruppe mit reduzierter Leistung auf niedriger Temperatur in der Größe von — 10 ºC bis — 25 ºC gehaltenes flüssiges Kühlmittel enthält.

9. Verfahren zur Sterilisation von Produkten in einer Anzahl von in einem Sterilisationsautoklaven nebeneinander angeordneten Körben, nach einem beliebigen der Ansprüche 1 bis 8, durch Gas oder Gas/Dampf-Mischung, der einen Behäl-

ter mit horizontaler Achse besitzt, bei dem ein Gas oder eine Gas/Dampf-Mischung im Behälter des Autoklaven behandelt wird und das Gas oder die Gas/Dampf-Mischung in Zirkulation mitgeführt wird, dadurch gekennzeichnet, daß das Gas oder die Mischung in Zirkulation ausschließlich quer zur Achse des Behälters folgend einem einzigen geschlossenen Kreislauf mitgeführt wird.

10. Verfahren nach Anspruch 9, dadurch gekennzeichnet, daß eine Gas/Dampf-Mischung in den Behälter durch Zuführung von Dampf in der Zirkulationsrichtung gebildet wird.

11. Verfahren nach einem der Ansprüche 9 bis 10, dadurch gekennzeichnet, daß das Gas oder die Gas/Dampf-Mischung mittels eines in dem Kreislauf angeordneten Austauschers erwärmt wird.

12. Verfahren zur Sterilisation nach einem beliebigen der Ansprüche 9 bis 11, außerdem dadurch gekennzeichnet, daß eine Kühlung durch Zerstäubung von kaltem Wasser sichergestellt ist, die horizontal in der Zirkulationsrichtung der Gas/Dampf-Mischung stromaufwärts der zu sterilisierenden Produkte orientiert ist.

# Fig.1

# Fig.2